# EUROPEAN PATENT SPECIFICATION

(11) **EP 3 151 901 B1**
(45) Date of publication and mention of the grant of the patent: **18.02.2026**
(21) Application number: 15802872.0
(22) Date of filing: 03.06.2015
(51) Int. Cl.: A61M 25/00, A61B 17/3207, A61M 25/01, A61B 17/22

(54) **INTRAVASCULAR CATHETER WITH DRUG DELIVERY SYSTEM**
INTRAVASKULÄRER KATHETER MIT WIRKSTOFFFREISETZUNGSSYSTEM
CATHÉTER INTRAVASCULAIRE AYANT UN SYSTÈME D'ADMINISTRATION DE MÉDICAMENT

(30) Priority: 03.06.2014 US 201462007039 P
(43) Date of publication of application: 12.04.2017
(73) Proprietor: VentureMed Group, Inc., Plymouth, MN 55441 (US)
(72) Inventor: Pigott, John P., Sylvania, OH 43560 (US)
(74) Representative: Haseltine Lake Kempner LLP
(86) International application number: PCT/US2015/033995
(87) International publication number: WO 2015/187838

(56) References cited:
- WO-A1-2013/159066
- US-A1- 2012 053 485
- US-A1- 2013 066 346
- US-B1- 6 283 947
- US-B1- 6 599 267
- US-B1- 6 692 466
- US-B2- 7 691 086
- US-B2- 8 366 661

## Description

### CROSS-REFERENCE TO RELATED APPLICATIONS

This application claims the benefit of U.S. Provisional Application No. 62/007,039, filed June 3, 2014.

### TECHNICAL FIELD

Embodiments of the present invention generally relate to an intravascular catheter device.

### BACKGROUND

Intravascular catheter devices can be used in minimally invasive surgical procedures, such as for treatment of atherosclerosis. Atherosclerosis is a chronic condition in which atheromatous plaque accumulates on the inner walls of a blood vessel. As a result, the blood vessel walls can become inflamed and, over time, may harden to form atherosclerotic lesions that cause a narrowing of the vessel lumen. In severe cases, the atherosclerotic lesions can rupture and induce the formation of thrombus (i.e., blood clots), which can prevent blood flow through the narrowed vessel lumen.

There are known procedures and devices for treating or otherwise reducing the risks associated with atherosclerosis. For example, an angioplasty is a procedure in which a balloon catheter is inserted into a narrowed region of the vessel lumen via a delivery catheter. The balloon catheter includes a flexible tube having an inflatable balloon at an end thereof. Once positioned in the narrowed region, the balloon is inflated in order to dilate the narrowed vessel lumen. The pressure in the balloon is generally sufficient to compress the accumulated plaque.

However, in some cases the treatment of atherosclerotic material can be accomplished or improved by the use of certain therapeutic agents (hereinafter also "drugs") such as, but not limited to, anti proliferative agents, immunosuppressive agents, angiopeptin, corticosteroids, prohelaing agents, and the like, at the site of stenosis. Therefore, it would be desirable to provide an intravascular catheter device having a drug delivery system capable of delivering drugs at the site of the atherosclerotic plaque accumulation.

There are other situations in which intravascular drug delivery is advantageous. Intravascular drug delivery permits the physician to precisely control the amount and location of drug administered to the patient. A smaller dosage may therefore be required for the drug to be effective, since no drug is lost by dilution, non-absorption, misdelivery, or other complications. Thereby minimizing costs and side effects. Additionally, intravascular delivery permits the drug to be delivered directly to a treatment site. This allows the effect of the drugs to be localized, which may limit or eliminate harmful side effects.

There are many other situations in which the delivery of drugs directly to a treatment site is advantageous. For example and not to serve as a limitation, in the case of renal hypertension it is sometimes advantageous to dennervate the tissue surrounding the renal artery. This may be accomplished chemically by introducing drugs directly to the site. Therefore, it is advantageous to have an intravascular drug delivery system capable of introducing drugs directly to a treatment site. Publication No. WO2013159066 provides a device for expanding tissue comprising at least one fluid delivery tube and at least one fluid delivery element in fluid communication with the at least one fluid delivery tube. US6599267 provides a catheter including an injection port at or near the distal end thereof and a mechanism for causing the injection port to move between a first position and a second position. US8366661 provides a medical device comprising a tubular catheter and an inner expandable body (e.g., a scaffolding) contained within the catheter. The inner expandable body is connected to a core wire for advancing or retracting the inner body. US7691086 provides a positionable, direct-injection catheters that can access a specific region of the heart or other organ. The catheter is provided with one or two needle shafts, which may be located within respective sheaths that extend axially along the interior of the lumen of a main catheter shaft. US Publication No. 2013066346 provides an intravascular catheter device for use during a surgical procedure. US6283947 provides a catheter for injecting medication to a specific point within a patient comprising a drug delivery lumen extending from a proximal end of the catheter to an injection port. US Publication No. 2012053485 provides a catheter including a catheter shaft, an expandable member coupled to an outer surface of the catheter shaft, and a needle having a distal end portion disposed on an outer side of the catheter shaft adjacent the expandable member. US Publication No. 6692466 provides a catheter assembly having a balloon disposed at the distal end thereof and a therapeutic substance delivery assembly, including a needle in fluid communication with a therapeutic substance delivery lumen for allowing a therapeutic substance to be injected into a diseased physiological lumen.

### SUMMARY OF THE INVENTIVE CONCEPT

This invention relates to an intravascular catheter device as set out in claim 1 below.

Optional features of the invention are set out in the dependent claims.

### BRIEF DESCRIPTION OF THE DRAWINGS

In addition to the features mentioned above, other aspects of the present invention will be readily apparent from the following descriptions of the drawings and exemplary embodiments, wherein like reference numerals across the several views refer to identical or equivalent features, and wherein:
**FIGURE 1** is a side elevation view of an intravascular catheter that includes a device in accordance with this invention for delivering drugs and other materials to a specific site within a blood vessel.
**FIGURE 2** is an enlarged side elevation view of the drug delivery portion of the intravascular catheter illustrated in Fig. 1 wherein the expandable portion is illustrated in the closed position and the drug delivery system is illustrated in the retracted position.
**FIGURE 3** is an end elevation view of the drug delivery portion of the intravascular catheter illustrated in Figs. 1 and 2 shown disposed within a blood vessel.
**FIGURE 4** is a side elevation view of the drug delivery portion of the first embodiment of the intravascular catheter illustrated in Figs. 1 through 3 wherein the expandable portion is illustrated in the open position and the drug delivery system is illustrated in the retracted position.
**FIGURE 5** is an end elevation view of the drug delivery portion of the intravascular catheter illustrated in Fig. 4.
**FIGURE 6** is a side elevation view of the drug delivery portion of the intravascular catheter illustrated in Figs. 1 through 5 wherein the expandable portion is illustrated in the open position and the drug delivery system is illustrated in the delivery position.
**FIGURE 7** is an end elevation view of the drug delivery portion of the intravascular catheter illustrated in Fig. 6.
**FIGURE 8** is an end elevation view of another exemplary embodiment of the intravascular catheter device in accordance with this invention, wherein the expandable portion is illustrated in the open position and the drug delivery system is illustrated in the delivery position.
**FIGURE 9** is an enlarged side view of a catheter tube having an expandable incising portion, in accordance with another exemplary embodiment of this invention.
**FIGURE 10** is a side view of the catheter tube shown in Fig. 9 illustrating the expandable incising portion in an opened position and the drug delivery device in the retracted position.
**FIGURE 11** is a side view of the catheter tube shown in Fig. 10 illustrating the expandable incising portion in an opened position and the drug delivery device in the delivery position.
**FIGURE 12** is an end view of the catheter tube as shown in Fig. 11.

### DETAILED DESCRIPTION OF EXEMPLARY EMBODIMENT(S)

The invention is described more fully hereinafter with reference to the accompanying drawings, in which exemplary embodiments of the invention are shown. This invention may, however, be embodied in many different forms and should not be construed as limited to the exemplary embodiments set forth herein. Rather, these embodiments are provided so that this disclosure will be thorough and complete, and will fully convey the scope of the invention to those skilled in the art. In the drawings, the size and relative sizes of layers and regions may be exaggerated for clarity.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/ or components, but do not preclude the presence or addition of one or more other features, integers, steps, operations, elements, components, and/or groups thereof.

Embodiments of the invention are described herein with reference to illustrations that are schematic illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

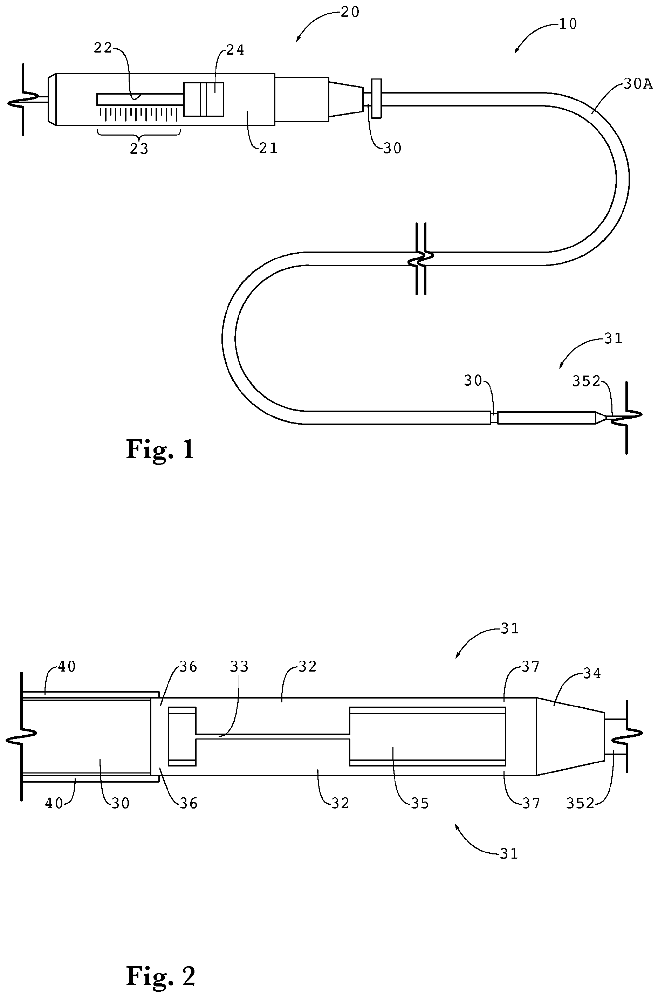

**FIGURE 1** illustrates an intravascular catheter 10 that includes a device in accordance with this invention for delivering drugs and other materials to a specific site within a blood vessel. The illustrated catheter 10 includes a handle assembly 20 having an elongated, cylindrical body 21. However, the handle assembly body 21 may have any desired shape and may be from any suitable material including, but not limited to, stainless steel or polymers. The illustrated handle assembly body 21 includes an internal chamber and a passageway (not shown) that extends through an end portion of the handle body 21 and is in communication with the internal chamber. The handle assembly body 21 further includes a slot 22 that extends through a side wall thereof and communicates with the internal chamber. One or more indicia 23 may be provided on the exterior of the handle assembly body 21 adjacent to the slot 22. A control member 24 is slidably disposed within the slot 22 adjacent to the indicia 23 for a purpose that will be explained below. If desired, a locking mechanism (not shown) may be provided to selectively retain the control member 25 in a desired position along the length of the slot 22, again for a purpose that will be explained below.

A catheter tube assembly 30 extends from the handle assembly 20. The catheter tube 330 may rest atop a guide wire 352. The guide wire 352 may extend through the catheter device 10. The illustrated catheter tube assembly 30 is an elongated, flexible member having a first end that is secured to the handle assembly 20 and a second end that is remote from the first end. The first end of the catheter tube assembly 30 may be secured to the handle assembly body 21 in any desired manner and communicate with the internal cavity through the passageway. The second end of the catheter tube assembly 30 may be secured to an expandable portion 31. The catheter tube assembly 30 may have any desired shape or size and may be made from any biocompatible material including, but not limited to, polyvinyl, polyethylene, nitinol, or stainless steel. A protective sheath 30a may be disposed about some or all of the catheter tube assembly 30.

The expandable portion may optionally comprise a sheath (not shown). The sheath may be configured to substantially surround the expandable portion. The sheath may also be configured to provide a watertight seal around the expandable portion, including the drug delivery device. Optionally, in such an embodiment the struts may be biased in the opened position, such that when the sheath is removed the struts automatically move into the opened position. In this embodiment, the struts may be comprised of any suitable material such as spring steel or the like.

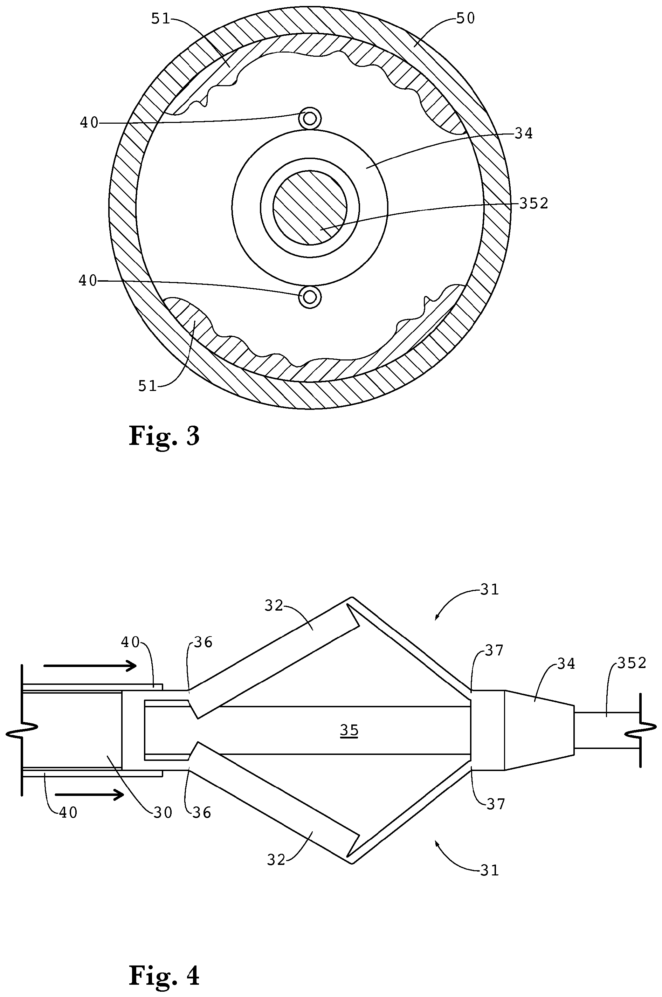

**FIGURE 2** and **FIGURE 3** illustrates the structure of the expandable portion 31 in detail. The second end of the catheter tube assembly 30 includes a radially outwardly expandable portion 31 that includes at least a pair of struts 32 that are separated by a longitudinally extending slit 33. Any number of struts 32 are contemplated. The illustrated catheter tube assembly 30 also includes a tip member 34 and a central inner sleeve 35.

First hinge portions 36 are respectively provided between the catheter tube assembly 30 and each of the struts 32. Similarly, second hinge portions 37 are respectively provided between each of the struts 32 and the tip member 34. Thus, the expandable portion 31 can be oriented in a closed position (illustrated in Figs. 1-3) or an opened position (illustrated in Figs. 4-7). In the closed position, the struts 32 are located radially inwardly toward one another and extend generally longitudinally along the inner sleeve 35. In the opened position, the struts 32 are located radially outwardly from one another and are angled outwardly away from the inner sleeve 35.

The expandable portion 31 of the second end of the catheter tube assembly 30 can be selectively moved between the closed position and the opened position by movement of the inner sleeve 35 relative to the catheter tube assembly 30. To accomplish this, the inner sleeve 35 extends from the second end of the catheter tube assembly 30 back to the first end thereof, where it is connected to the control member 24 (or any other desired structure that can be manipulated by a user of the device 10). Thus, movement of the control member 24 throughout the slot 22 causes corresponding movement of the inner sleeve 35 throughout the catheter assembly 10. In the illustrated embodiment, when the control member 24 is moved to a first position (farthest to the right in the slot 22 when viewing Fig. 1) relative to the handle assembly body 21, the inner sleeve 35 is extended so as to cause the expandable portion 31 to be moved to the closed position illustrated in Figs. 1 through 3. Conversely, when the control member 24 is moved to a second position (farthest to the left in the slot 22 when viewing Fig. 1) relative to the handle assembly body 21, the inner sleeve 35 is retracted so as to cause the expandable portion 31 to be moved to the opened position illustrated in Figs. 4 through 7.

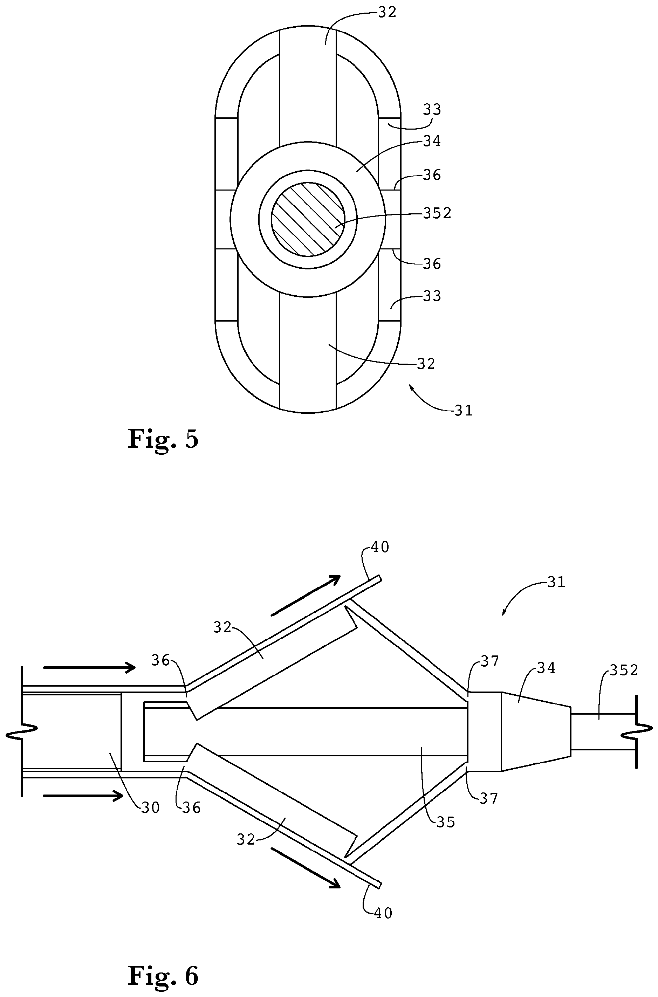

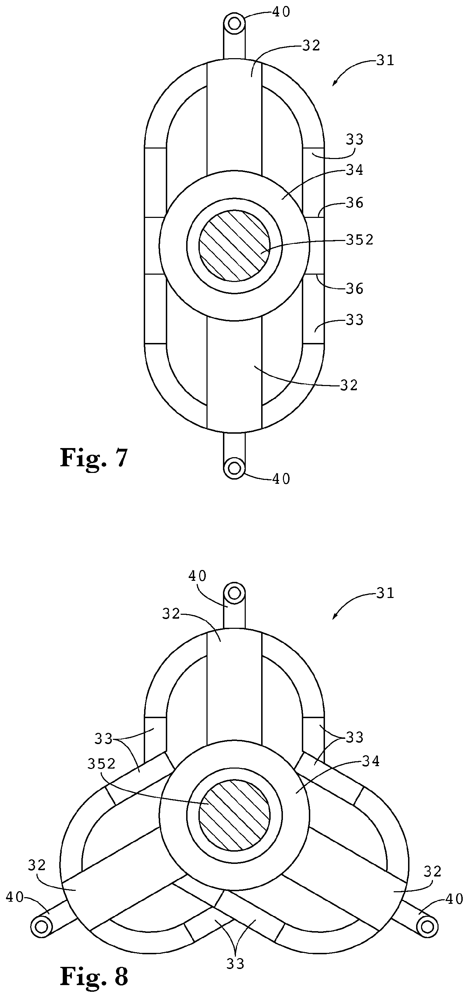

The expandable portion 31 further includes a mechanism for delivering drugs and other materials to a specific site within a blood vessel or other desired location within a human or animal body. This mechanism for delivering drugs and other materials may include a first and second drug delivery device 40. The drug delivery device 40 may be in the form of a tube, such as a needle. Each of the first and second drug delivery devices 40 extends from the first end of the catheter 10 to the second end thereof and is selectively movable relative thereto between a retracted position (illustrated in Figs. 2 through 5) and an extended position (illustrated in Figs. 6 and 7). The first ends of each of the first and second drug delivery devices 40 communicate with a source (not shown) of drugs and other materials in such a manner that the drugs and other materials can be supplied from the source of drugs and other materials through the first and second drug delivery devices 40 to the remote open ends thereof located at the second end of the catheter 10. The source may comprise, for example and not to serve as a limitation, a bladder located in the handle assembly 20.

The first and second drug delivery devices 40 can be selectively moved between the retracted and extended positions in any desired manner. Preferably, however, a mechanism (not shown) is provided on the handle assembly body 21 of the catheter 10 and can be manipulated by a user of the device 10 to selectively move the first and second drug delivery devices 40 between the retracted and extended positions. This movement-imparting mechanism may, for example, be embodied as an auxiliary control member that, similar to the control member 24, can be moved to position the first and second drug delivery devices 40 at any desired location. In the illustrated embodiment, the first and second drug delivery devices 40 are moved concurrently as a single unit. However, if desired, the first and second drug delivery devices 40 may be moved independently of one another, such as by providing separate auxiliary control members on the handle assembly body 21 of the catheter 10.

The same or an additional control member may be provided on the handle assembly 20 to control the dosage of drug delivered through the drug delivery devices 40. This additional control member may be integrated as part of the aforementioned movement-imparting mechanism or may be a separate, third, control member. The handle assembly 20 may comprise a pair of additional indication devices that indicates the position of the drug delivery devices 40 and the dosage administered. Alternatively, these additional indication devices may be integrated into a single indication device. These additional indication devices may be similar to the indicia 23.

In use, the expandable portion 31 of the second end of the catheter 10 is initially operated to the closed position illustrated in Figs. 1 through 3. At the same time, the first and second drug delivery devices 40 are moved to the retracted positions illustrated in Figs. 1 through 5. When these initial orientations have been achieved, the second end of the catheter 10 can be inserted into a blood vessel 50 and disposed adjacent to an area of interest, such as a deposit of an atherosclerotic material 51, for example, as shown in Fig. 2. In exemplary embodiments, the guide wire 352 may be initially inserted, and the catheter 10 may be inserted atop of the guide wire 352 to be guided to the area of interest.

Referring now to **FIGURE 4** and **FIGURE 5****,** once the second end of the catheter 10 is disposed adjacent to the area of interest, the expandable portion 31 is then operated to move from the closed position to the opened position by movement of the inner sleeve 35 relative to the catheter tube assembly 30. As discussed above, in the opened position, the struts 32 are located radially outwardly from one another and are angled outwardly away from the inner sleeve 35. Thus, as best shown in Fig. 4, the struts 32 present outwardly-extending ramped surfaces adjacent to the leading ends of the first and second drug delivery devices 40.

As illustrated in **FIGURE 6** and **FIGURE 7**, thereafter, the movement-imparting mechanism is moved so as to extend the first and second drug delivery devices 40 from the retracted positions to the extended positions. When so extended, the first and second drug delivery devices 40 initially slide longitudinally along the catheter tube assembly 30 until the leading ends thereof engage the outwardly-extending ramped surfaces of the extended struts 32. At that point, the leading ends of the first and second drug delivery devices 40 slide outwardly along outwardly-extending ramped surfaces of the extended struts 32. The leading ends of the first and second drug delivery devices 40 may be moved outwardly past the ends of the extended struts 32 either adjacent to or into engagement with the deposit of an atherosclerotic material 51 within the blood vessel 50 or any other area of interest. In this manner, the drugs and other materials can be supplied from the source of drugs and other materials, optionally via a dosage control mechanism (not shown), through the first and second drug delivery devices 40 to the remote open ends thereof located at the second end of the catheter 10.

If desired, the leading ends of the first and second drug delivery devices 40 may be moved outwardly past the ends of the extended struts 32 into engagement with the blood vessel 50 and the surrounding tissue so that the drugs and other materials can be supplied either to a specific site inside of a blood vessel, within the blood vessel itself, or to the surrounding tissue outside of the blood vessel.

**FIGURE 8** illustrates another exemplary embodiment comprising three struts 32. The expandable portion 31 of the second end of the catheter 10 may be provided with three (or more) struts 32, each having a single drug delivery device 40 associated therewith.

It is notable that Fig. 1 though Fig.7 illustrates the expandable portion 31 of the catheter 10 provided with two struts 32 that are oriented on opposite sides of the second end thereof, i.e., at an 180 degree orientation relative to one another. However, it will be appreciated that the struts 32 may be alternatively oriented at any other desired angle relative to one another. Furthermore, the expandable portion 31 of the second end of the catheter 10 may be provided with a greater or lesser number of such struts 32. For example, the expandable portion 31 of the second end of the catheter 10 may be provided with only a single strut 32 having a single drug delivery device 40 associated therewith.

Lastly, in the illustrated first embodiment of this invention, the expandable portion 31 of the second end of the catheter 10 is provided with two struts 32 that are respectively provided with single drug delivery device 40. However, it will be appreciated that some or all of the struts 32 may be provided with two or more of the drug delivery devices 40 as desired.

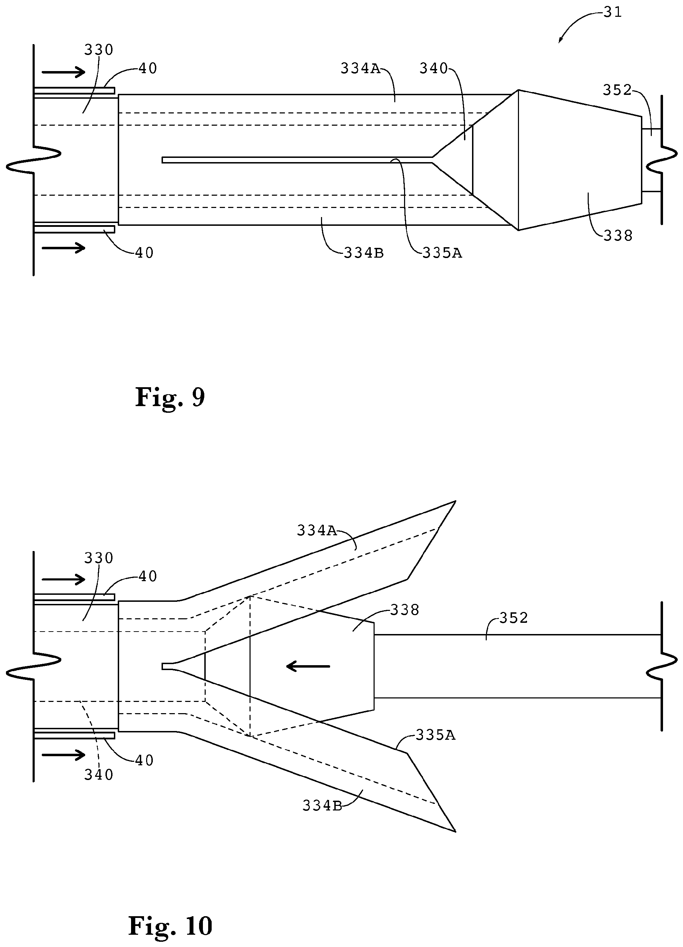

**FIGURE 9** illustrates the catheter tube 330 having an expandable portion 31, in accordance with another exemplary embodiment of this invention. The catheter tube 330 may extend from a handle assembly (not shown) as described above in Fig. 1. The catheter tube 330 may rest atop the guide wire 352. The guide wire 352 may extend through the catheter device 10. The expandable portion 31 is provided on the distal end of the catheter tube 330 and may include a tip member 338. The catheter tube 330 may also include an inner sleeve 340 that is attached to the tip member 338 and a protective sheath (not shown), which is also described above in Fig. 1.

In the illustrated embodiment, the expandable portion 31 includes a pair of struts 334A and 334B that are supported thereon in a cantilevered manner (i.e., not attached to one another or to the tip member 338 at their distal ends), the purpose of which will be explained below. The struts 334A and 334B are separated by a pair of longitudinally extending slits 335A and 335B (335B not shown) that extend from the end of the expandable portion 31. It should be appreciated, however, that the expandable portion 31 may have any number or configuration of struts, corresponding longitudinally extending slits, and incising elements as desired.

The illustrated struts 334A and 334B are supported on the expandable portion 31 so that they can be splayed open in a Y shaped configuration. For example, the struts 334A and 334B may be splayed open by drawing the inner sleeve 340 within the catheter tube 330, as previously described in other embodiments. In doing so, the tip member 338 slides along the inner surfaces of the struts 334A and 334B and pivots them outwardly. Alternatively (or in addition), the struts 334A and 334B may be biased in the splayed open position. In such an embodiment, the protective sheath (not shown) may be used to effect movement of the expandable portion 31 between a closed position and the splayed open position.

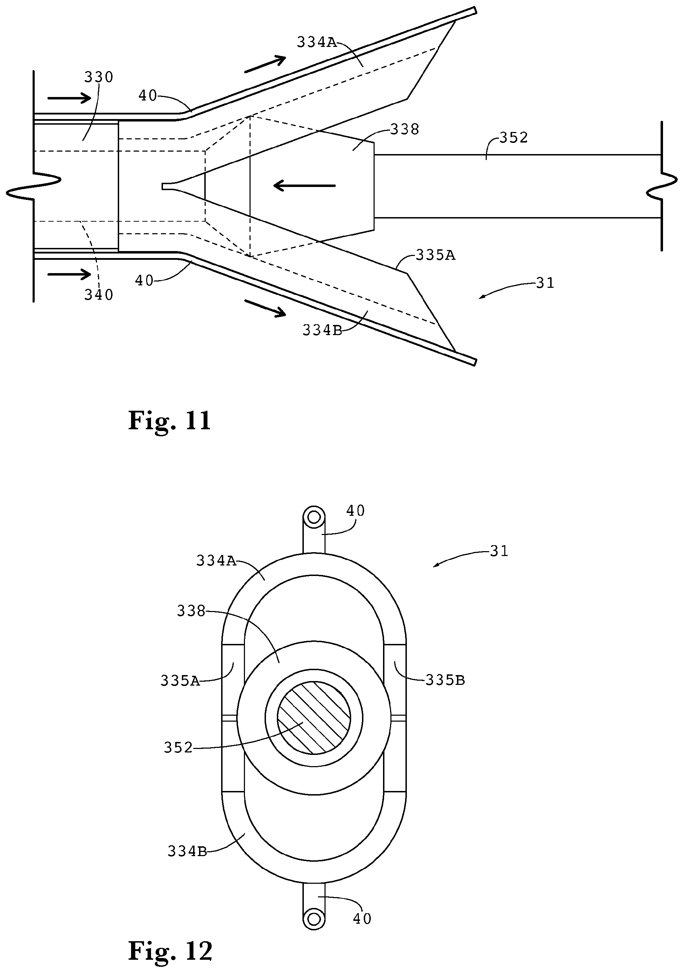

The expandable portion 31 further includes a mechanism for delivering drugs and other materials to a specific site within a blood vessel or other desired location within a human or animal body. This mechanism for delivering drugs and other materials may include a first and second drug delivery device 40. The drug delivery device 40 may be in the form of a tube. Alternatively, the drug delivery device 40 may be a needle or a syringe. Each of the first and second drug delivery devices 40 extends from the first end of the catheter 10 to the second end thereof and is selectively movable relative thereto between a retracted position (illustrated in Fig. 9 and Fig. 10) and an extended position (illustrated in Figs. 11 and 12). The first ends of each of the first and second drug delivery devices 40 may be in communication with a source (not shown) of drugs and other materials in such a manner that the drugs and other materials can be supplied from the source of drugs and other materials through the first and second drug delivery devices 40 to the remote open ends thereof located at the second end of the catheter 10. The source may comprise, for example and not to serve as a limitation, a bladder located in the handle assembly 20.

The first and second drug delivery devices 40 may be selectively moved between the retracted and extended positions in any desired manner. Preferably, however, a mechanism (not shown) is provided on the handle assembly body 21 of the catheter 10 and can be manipulated by a user of the device 10 to selectively move the first and second drug delivery devices 40 between the retracted and extended positions. This movement-imparting mechanism may, for example, be embodied as an auxiliary control member that, similar to the control member 24, can be moved to position the first and second drug delivery devices 40 at any desired location. In the illustrated embodiment, the first and second drug delivery devices 40 are moved concurrently as a single unit. However, if desired, the first and second drug delivery devices 40 may be moved independently of one another, such as by providing separate auxiliary control members on the handle assembly body 21 of the catheter 10.

The same or an additional control member may be provided on the handle assembly 20 to control the dosage of drug delivered through the drug delivery devices 40. This additional control member may be integrated as part of the aforementioned movement-imparting mechanism or may be a separate, third, control member. The handle assembly 20 may comprise a pair of additional indication devices that indicates the position of the drug delivery devices 40 and the dosage administered. Alternatively, these additional indication devices may be integrated into a single indication device. These additional indication devices may be similar to the indicia 23.

In use, the expandable portion 31 of the second end of the catheter 10 is initially operated to the closed position illustrated in Fig. 9. At the same time, the first and second drug delivery devices 40 are moved to the retracted positions illustrated in Fig. 9. When these initial orientations have been achieved, the second end of the catheter 10 can be inserted into a blood vessel 50 and disposed adjacent to an area of interest, such as a deposit of an atherosclerotic material 51, for example, as shown in Fig. 2. In exemplary embodiments, the guide wire 352 may be initially inserted, and the catheter 10 may be inserted atop of the guide wire 352 to be guided to the area of interest.

Referring now to **FIGURE 10**, once the second end of the catheter 10 is disposed adjacent to the area of interest, the expandable portion 31 is then operated to move from the closed position to the opened position by movement of the inner sleeve 340 relative to the catheter tube assembly 330. As discussed above, in the opened position, the struts 334A and 334B are located radially outwardly from one another and are angled outwardly away from the inner sleeve 340. Thus, the struts 334A and 334B present outwardly-extending ramped surfaces adjacent to the leading ends of the first and second drug delivery devices 40.

Thereafter, as illustrated in **FIGURE 11** and **FIGURE 12** the movement-imparting mechanism is moved so as to extend the first and second drug delivery devices 40 from the retracted positions, as illustrated in Fig. 11, to the extended positions, as illustrated in Fig. 12. When so extended, the first and second drug delivery devices 40 initially slide longitudinally along the catheter tube assembly 330 until the leading ends thereof engage the outwardly-extending ramped surfaces of the extended struts 334A and 334B. At that point, the leading ends of the first and second drug delivery devices 40 slide outwardly along outwardly-extending ramped surfaces of the extended struts 334A and 334B. The leading ends of the first and second drug delivery devices 40 may be moved outwardly past the ends of the extended struts 334A and 334B either adjacent to or into engagement with the deposit of an atherosclerotic material 51 within the blood vessel 50 or any other area of interest. In this manner, the drugs and other materials can be supplied from the source of drugs and other materials, optionally via the dosage control mechanism, through the first and second drug delivery devices 40 to the remote open ends thereof located at the second end of the catheter 10.

If desired, the leading ends of the first and second drug delivery devices 40 may be moved outwardly past the ends of the extended struts 334A and 334B into engagement with the blood vessel 50 and the surrounding tissue so that the drugs and other materials can be supplied either to a specific site inside of a blood vessel, within the blood vessel itself, or to the surrounding tissue outside of the blood vessel.

Those skilled in the art will realize that many variations and modifications may be made to affect the described invention and still be within the scope of the claimed invention. Additionally, many of the elements indicated above may be altered or replaced by different elements which will provide the same result. It is the intention, therefore, to limit the invention only as indicated by the scope of the claims.

## Claims

1. An intravascular catheter device comprising:
a catheter tube (30) having a first end and a second end;
a selectively expandable portion (31) secured to said second end of said catheter tube (31) and having a plurality of struts (32) each defining an outer surface, wherein the expandable portion (31) is operable between a closed position where the expandable portion (31) has a first diameter, and an opened position where the expandable portion (31) has a second diameter that is larger than the first diameter;
at least one drug delivery device (40) configured to deliver a therapeutic agent, wherein the drug delivery device (40) is selectively operable between a retracted position where the drug delivery device (40) extends along the catheter tube (30), and a delivery position where the drug delivery device (40) additionally extends along an outer surface of, and beyond, one of the struts (32);
a handle assembly (20) attached to said first end of said catheter tube (30) and
comprising a control member (24) movably supported thereon for controlling operation of the expandable portion (31);
an inner sleeve (35) moveably mounted within the catheter tube (30), wherein a first end of the inner sleeve (35) extends from, and is in an actuatable relationship with, the control member (24) of the handle assembly (20) and a second end of the inner sleeve (35) is connected with a tip member (34), such that actuation of said control member (24) causes movement of the inner sleeve (35) and tip member (34) relative to the catheter tube (30), thereby causing the expandable portion (31) to move between the closed position and the opened position.

2. The intravascular catheter device of Claim 1, wherein:
the drug delivery device (40) is a needle.

3. The intravascular catheter device of Claim 1, wherein:
the at least one drug delivery device (40) comprises a plurality of the drug delivery devices (40); and
one of the plurality of the drug delivery devices (40) is located on each of the plurality of struts (32).

4. The intravascular catheter device of claim 1, wherein:
the handle assembly (20) further comprises a bladder for storing the therapeutic agent.

5. The intravascular catheter device of claim 4 wherein:
the drug delivery device (40) is in communication with the handle assembly (20).

6. The intravascular catheter device of claim 5, wherein:
the handle assembly (20) further comprises an indicator device which displays the position of the expandable portion (31).

7. The intravascular catheter device of Claim 1, further comprising:
a protective sheath surrounding and forming a watertight seal around the expandable portion (31).

8. The intravascular catheter device of Claim 7, wherein:
each of the plurality of struts (32) are biased in the opened position.

9. The intravascular catheter device of any of the preceding claims, wherein:
each of the plurality of struts (32) are configured to present outwardly-extending ramped surfaces adjacent to leading ends of the first and second drug delivery devices (40) when the expandable portion (32) is placed in the opened position.

10. The intravascular catheter device of any of the preceding claims, further comprising:
a first hinge portion (36) provided between the catheter tube (30) and each of the plurality of struts (32); and
a second hinge portion (37) provided between each the plurality of struts (32) and
the tip member (34);
wherein each of said plurality of struts (32) is configured to additionally hinge about substantially a midpoint thereof.

11. The intravascular catheter device of any of the preceding claims, wherein:
the catheter device is configured to receive a guidewire which extends through the catheter device.

12. The intravascular catheter device of any of the preceding claims, wherein:
said drug delivery device (40) extends entirely outside of said one of the struts (32).

## Patentansprüche

1. Intravaskuläre Kathetervorrichtung, umfassend:
einen Katheterschlauch (30), der ein erstes Ende und ein zweites Ende aufweist;
einen selektiv aufweitbaren Abschnitt (31), der an dem zweiten Ende des Katheterschlauchs (31) befestigt ist und eine Vielzahl von Streben (32) aufweist, wobei jede eine Außenfläche definiert, wobei der aufweitbare Abschnitt (31) zwischen einer geschlossenen Position, in welcher der aufweitbare Abschnitt (31) einen ersten Durchmesser aufweist, und einer geöffneten Position, in welcher der aufweitbare Abschnitt (31) einen zweiten Durchmesser aufweist, der größer als der erste Durchmesser ist, bedienbar ist;
mindestens eine Wirkstofffreisetzungsvorrichtung (40), die dazu konfiguriert ist, ein Arzneimittel freizusetzen, wobei die Wirkstofffreisetzungsvorrichtung (40) selektiv zwischen einer zurückgezogenen Position, in der sich die Wirkstofffreisetzungsvorrichtung (40) entlang des Katheterschlauchs (30) erstreckt, und einer Freisetzungsposition, in der sich die Wirkstofffreisetzungsvorrichtung (40) zusätzlich entlang einer Außenfläche einer der Streben (32) und über diese hinaus erstreckt, betreibbar ist;
eine Griffbaugruppe (20), die an dem ersten Ende des Katheterschlauchs (30) angebracht ist und
ein Steuerelement (24) umfasst, das beweglich darauf gelagert ist, um den Betrieb des aufweitbaren Abschnitts (31) zu steuern;
eine Innenhülse (35), die bewegbar innerhalb des Katheterschlauchs (30) montiert ist, wobei sich ein erstes Ende der Innenhülse (35) von dem Steuerelement (24) der Griffbaugruppe (20) erstreckt und mit diesem in einer betätigbaren Beziehung steht und ein zweites Ende der Innenhülse (35) mit einem Spitzenelement (34) verbunden ist, sodass eine Betätigung des Steuerelements (24) eine Bewegung der Innenhülse (35) und des Spitzenelements (34) relativ zu dem Katheterschlauch (30) bewirkt, wodurch bewirkt wird, dass sich der aufweitbare Abschnitt (31) zwischen der geschlossenen Position und der geöffneten Position bewegt.

2. Intravaskuläre Kathetervorrichtung nach Anspruch 1, wobei:
die Wirkstofffreisetzungsvorrichtung (40) eine Nadel ist.

3. Intravaskuläre Kathetervorrichtung nach Anspruch 1, wobei:
die mindestens eine Wirkstofffreisetzungsvorrichtung (40) eine Vielzahl von Wirkstofffreisetzungsvorrichtungen (40) umfasst; und
sich eine der Vielzahl von Wirkstofffreisetzungsvorrichtungen (40) an jeder der Vielzahl von Streben (32) befindet.

4. Intravaskuläre Kathetervorrichtung nach Anspruch 1, wobei:
die Griffbaugruppe (20) ferner eine Blase zum Lagern des Arzneimittels umfasst.

5. Intravaskuläre Kathetervorrichtung nach Anspruch 4, wobei:
die Wirkstofffreisetzungsvorrichtung (40) mit der Griffbaugruppe (20) in Verbindung steht.

6. Intravaskuläre Kathetervorrichtung nach Anspruch 5, wobei:
die Griffbaugruppe (20) ferner eine Indikatorvorrichtung umfasst, welche die Position des aufweitbaren Abschnitts (31) anzeigt.

7. Intravaskuläre Kathetervorrichtung nach Anspruch 1, ferner umfassend:
eine Schutzhülle, die den aufweitbaren Abschnitt (31) umgibt und eine wasserdichte Abdichtung um diesen herum bildet.

8. Intravaskuläre Kathetervorrichtung nach Anspruch 7, wobei:
jede der Vielzahl von Streben (32) in der geöffneten Position vorgespannt ist.

9. Intravaskuläre Kathetervorrichtung nach einem der vorhergehenden Ansprüche, wobei:
jede der Vielzahl von Streben (32) dazu konfiguriert ist, nach außen verlaufende, rampenförmige Flächen benachbart zu vorderen Enden der ersten und zweiten Wirkstofffreisetzungsvorrichtung (40) darzustellen, wenn der aufweitbare Abschnitt (32) in der geöffneten Position angeordnet ist.

10. Intravaskuläre Kathetervorrichtung nach einem der vorhergehenden Ansprüche, ferner umfassend:
einen ersten Scharnierabschnitt (36), das zwischen dem Katheterschlauch (30) und jeder der Vielzahl von Streben (32) bereitgestellt ist; und
ein zweiter Scharnierabschnitt (37), der zwischen jeder der Vielzahl von Streben (32) und
dem Spitzenelement (34) bereitgestellt ist;
wobei jede der Vielzahl von Streben (32) dazu konfiguriert ist, zusätzlich um im Wesentlichen einen Mittelpunkt davon um ein Scharnier zu drehen.

11. Intravaskuläre Kathetervorrichtung nach einem der vorhergehenden Ansprüche, wobei:
die Kathetervorrichtung dazu konfiguriert ist, einen Führungsdraht aufzunehmen, der sich durch die Kathetervorrichtung zu erstrecken.

12. Intravaskuläre Kathetervorrichtung nach einem der vorhergehenden Ansprüche, wobei:
sich die Wirkstofffreisetzungsvorrichtung (40) vollständig außerhalb einer der Streben (32) erstreckt.

## Revendications

1. Dispositif de cathéter intravasculaire comprenant :
un tube de cathéter (30) présentant une première extrémité et une seconde extrémité ;
une partie extensible (31) de manière sélective fixée à ladite seconde extrémité dudit tube de cathéter (31) et présentant une pluralité d'entretoises (32) définissant chacune une surface externe, dans lequel la partie extensible (31) peut fonctionner entre une position fermée où la partie extensible (31) présente un premier diamètre, et une position ouverte où la partie extensible (31) présente un second diamètre qui est plus grand que le premier diamètre ;
au moins un dispositif d'administration de médicament (40) configuré pour administrer un agent thérapeutique, dans lequel le dispositif d'administration de médicament (40) peut fonctionner de manière sélective entre une position rétractée où le dispositif d'administration de médicament (40) s'étend le long du tube de cathéter (30), et une position d'administration où le dispositif d'administration de médicament (40) s'étend en outre le long d'une surface externe et au-delà de l'une des entretoises (32) ;
un ensemble de poignée (20) rattaché à ladite première extrémité dudit tube de cathéter (30) et
comprenant un élément de commande (24) supporté de manière mobile sur celui-ci pour commander le fonctionnement de la partie extensible (31) ;
un manchon interne (35) monté de manière mobile à l'intérieur du tube de cathéter (30), dans lequel une première extrémité du manchon interne (35) s'étend à partir de l'élément de commande (24) de l'ensemble de poignée (20) et est en relation d'actionnement avec celui-ci, et une seconde extrémité du manchon interne (35) est reliée à un élément de pointe (34), de sorte que l'actionnement dudit élément de commande (24) provoque le mouvement du manchon interne (35) et de l'élément de pointe (34) par rapport au tube de cathéter (30), provoquant ainsi le déplacement de la partie extensible (31) entre la position fermée et la position ouverte.

2. Dispositif de cathéter intravasculaire selon la revendication 1, dans lequel :
le dispositif d'administration de médicament (40) est une aiguille.

3. Dispositif de cathéter intravasculaire selon la revendication 1, dans lequel :
l'au moins un dispositif d'administration de médicament (40) comprend une pluralité des dispositifs d'administration de médicament (40) ; et
l'un de la pluralité des dispositifs d'administration de médicaments (40) est situé sur chacune des entretoises (32).

4. Dispositif de cathéter intravasculaire selon la revendication 1, dans lequel :
l'ensemble de poignée (20) comprend également une vessie de stockage de l'agent thérapeutique.

5. Dispositif de cathéter intravasculaire selon la revendication 4, dans lequel :
le dispositif d'administration de médicament (40) est en communication avec l'ensemble de poignée (20).

6. Dispositif de cathéter intravasculaire selon la revendication 5, dans lequel :
l'ensemble de poignée (20) comprend également un dispositif indicateur qui affiche la position de la partie extensible (31).

7. Dispositif de cathéter intravasculaire selon la revendication 1, comprenant également :
une gaine protectrice entourant et formant un joint étanche autour de la partie extensible (31).

8. Dispositif de cathéter intravasculaire selon la revendication 7, dans lequel :
chacune de la pluralité d'entretoises (32) est précontrainte en position ouverte.

9. Dispositif de cathéter intravasculaire selon l'une quelconque des revendications précédentes, dans lequel :
chacune de la pluralité d'entretoises (32) est configurée pour présenter des surfaces en rampe s'étendant vers l'extérieur, adjacentes aux extrémités avant des premier et second dispositifs d'administration de médicament (40) lorsque la partie extensible (32) est placée dans la position ouverte.

10. Dispositif de cathéter intravasculaire selon l'une quelconque des revendications précédentes, comprenant également :
une première partie de charnière (36) prévue entre le tube de cathéter (30) et chacune de la pluralité d'entretoises (32) ; et
une seconde partie de charnière (37) prévue entre chacune de la pluralité d'entretoises (32) et
l'élément de pointe (34) ;
dans lequel chacune de ladite pluralité d'entretoises (32) est configurée pour s'articuler en outre autour sensiblement d'un point médian de celle-ci.

11. Dispositif de cathéter intravasculaire selon l'une quelconque des revendications précédentes, dans lequel :
le dispositif de cathéter est configuré pour recevoir un fil-guide s'étendant à travers le dispositif de cathéter.

12. Dispositif de cathéter intravasculaire selon l'une quelconque des revendications précédentes, dans lequel :
le dispositif d'administration de médicament (40) s'étend entièrement à l'extérieur de l'une desdites entretoises (32).
